# EUROPEAN PATENT SPECIFICATION

(11) **EP 0 597 018 B1**
(45) Date of publication and mention of the grant of the patent: **06.10.1999**
(21) Application number: 92917211.2
(22) Date of filing: 21.07.1992
(51) Int. Cl.: C07J 17/00, A61K 31/70, C07H 15/26, C07H 15/203, C07H 15/252, C07H 15/207, C07H 17/04, C07H 15/18, C07H 15/24

(54) **GLYCOSIDATION REACTION**
GLYKOSIDIERUNGSVERFAHREN
REACTION DE GLYCOSIDATION

(30) Priority: 22.07.1991 US 733915; 24.01.1992 US 815691
(43) Date of publication of application: 18.05.1994
(73) Proprietor: HARRIER, INC., Hermosa Beach, CA 90254 (US)
(72) Inventor: KLEMKE, R.Erich, D-7709 Hilzingen/Reidheim (DE); KOREEDA, Masata, Ann Arbor, MI 48103 (US)
(74) Representative: Moreland, David, Dr.
(86) International application number: US9206063
(87) International publication number: WO9301821

(56) References cited:
- WO-A-91/11452
- FR-A- 2 007 410
- US-A- 4 415 731
- JOURNAL OF THE AMERICAN CHEMICAL SOCIETY, vol.102, no.2, 16 January 1980, DC US pages 857 - 858 C. COLAS ET AL 'Synthesis of 2,5,6-trideoxystreptamine and its transformation into bioactive pseudodisaccharides by microbial and chemical methods.'
- Journal of Organic Chemistry, Vol. 55, issued 1990, V. BOLITT et al., "Direct Preparation of 2-Deoxy-D-Glucopyranosides from Glucals without Ferrier Rearrangement", pages 5812-5813, see entire document.
- Journal of the Chemical Society, issued 1969, R.J. FERRIER et al., "Unsaturated Carbohydrates. Part IX. Synthesis of 2,3-Dideoxy-alpha-D-erythro-hex-2-enopyrano sides from Tri-O-acetyl-D-glucal", pages 570-575, see entire document.
- Liebigs Annals of Chemistry, issued 1985, THIEM et al., "Untersuchungen zur Darstellung von Desoxyzucker-Steroidglycosiden", pages 2135-2150, see entire document.
- Advances in Carbohydrate Chemistry, Vol. 20, issued 1965, R.J. FERRIER, "Unsaturated Sugars", pages 67 and 90-93.
- The Journal of Organic Chemistry, Vol. 55, No. 1, issued 05 January 1990, S. RAMESH et al., "Aureolic Acid Antibiotics: A Simple Method for 2-Deoxy-beta-Glycosidation", pages 5-7, see entire document.
- THIEM, "Trends in Synthetic Carbohydrate Chemistry", Chapter 8, published 1989 by American Chemical Society, pages 131-149.
- Carbohydrate Research, Vol. 130, issued 1984, K. BOCK et al., "2-Bromo-2-Deoxy Sugars as Starting Materials for the Synthesis of alpha- or beta-Glycosides of 2-Deoxy Sugars", pages 125-134.

## Description

This invention relates to a surprisingly novel method for the production of a broadly novel type of glycoside. The method comprises glycosylation (i.e., glycosidation) of an aglycon compound having a functional group, e.g. a hydroxy compound such as a hydroxy-steroid or hydroxy-non-steroid.

J. THIEM et al Liebigs Ann Chem. 1985, 2135-2150 discloses an N-iodosuccinimide catalysed reaction of glycal with a steroid aglycon (cholesterin) the Fernier rearrangement taking place and a 2', 3' - unsaturated glycoside obtained.

C. COLAS et al JACS 102/2, 857 (1980) discloses chiral 2,5,6 - trideoxystreptamine as an aglycon, its synthesis from quinic acid and its microbial and chemical transformation into two bioactive pseudodisaccharides.

WO 91/11452 refers to the production of steroidal glycosides; this reference is relevant under Art 54(3)EPC.

There has been surprisingly found according to a first aspect of the present invention a method for the production of a pyranoside compound, wherein a aglycon compound selected from aliphatic, alicyclic, aliphatic-aromatic and aromatic compounds having a primary, secondary or tertiary functional group selected from -SH and -COOH is glycosylated, characterized in that the aglycon compound is reacted in a solvent with 3,4,6-trio-O-acyl-glucal of formula 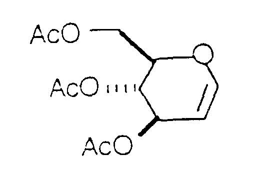 in the presence of molecular halogen preferably molecular iodine as a catalyst to produce the corresponding, 4,6-di-O-acyl-2,3-dideoxy-α-D-erythro-hex-2-enopyranoside of said aglycon compound, where acyl is a lower acyl group.

A steroidal glycoside -- a 3-β-ol cholesterol pyranoside which is cholesteryl 4,6-di-O-acetyl-2,3-dideoxy-α-D-erythro-hex-2-enopyranoside, obtainable by this method --has been found to be applicable as a pharmacologically active agent for use as a medicament, especially as an anti-neoplastic agent, or in geriatric medicine, or as a sedative or activity-enhancing agent. For convenience in describing the invention, the 4,6-di-O-acyl (or acetyl)-2,3-dideoxy-α-D-erythro-hex-2-enopyranoside will sometimes be referred to herein simply as a DDH pyranoside.

Further, there has been surprisingly found according to a second aspect of the present invention a method for the production of a pyranoside compound, wherein a aglycon compound selected from aliphatic, alicyclic, aliphatic-armoatic and aromatic compounds having a primary, secondary or tertiary functional group selected from -OH, -SH and - COOH is glycosylated, characterized in that the aglycon compound is reacted in tetrahydrofuran with 3,4,6-trio-O-acyl-glucal of formula 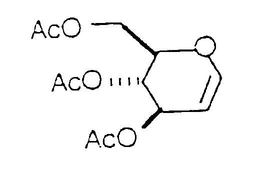 in the presence of molecular halogen preferably molecular iodine as a catalyst to produce the corresponding 4,6-di-O-acyl-2,3-dideoxy-α-D-erythro-hex-2-enopyranoside of said algycon compound, where acyl is a lower acyl group.

In the accompanying drawings with reference to preferred examples of the invention:
FIGURE 1 is an infrared spectrum of the glucal used in the reaction of Example 1;
FIGURE 2 is an infrared spectrum of the glycosylation product of Example 1;
FIGURE 3 is an NMR spectrum of the same glycosylation product of Example 1;

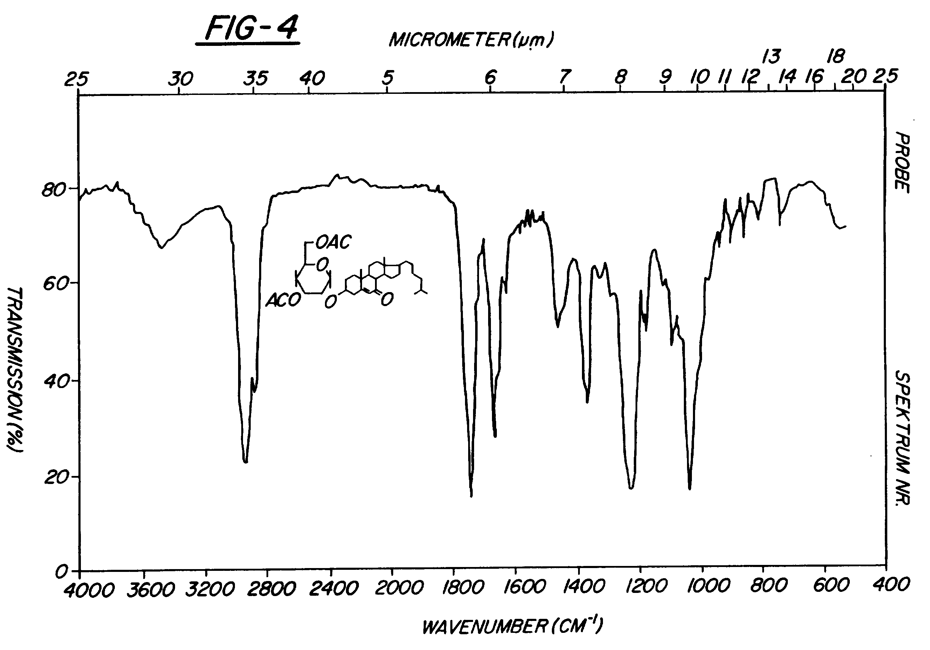

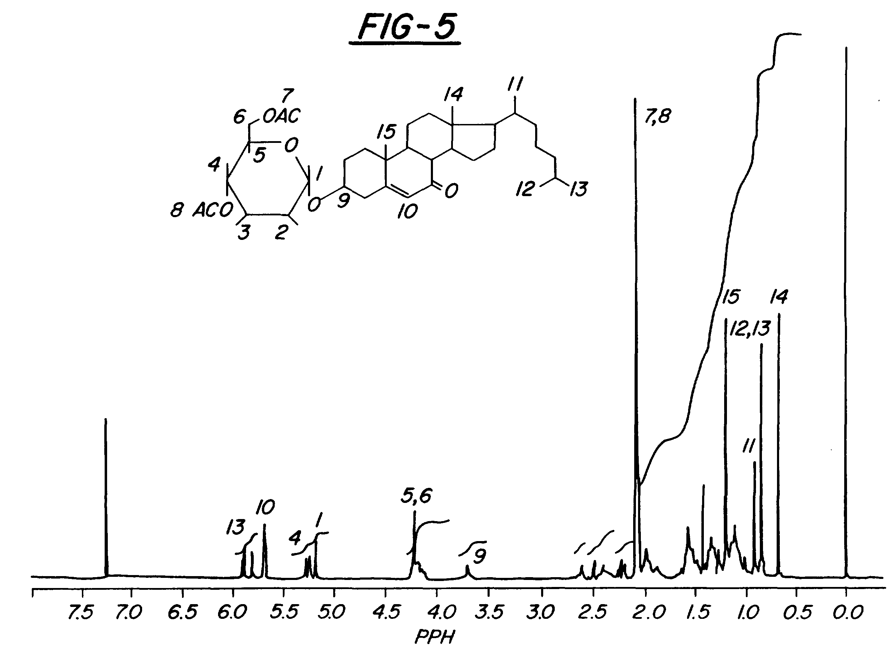

FIGURES 4 and 5 are the IR-spectrum and the NMR-spectrum, respectively of the ketone product of Example 2;

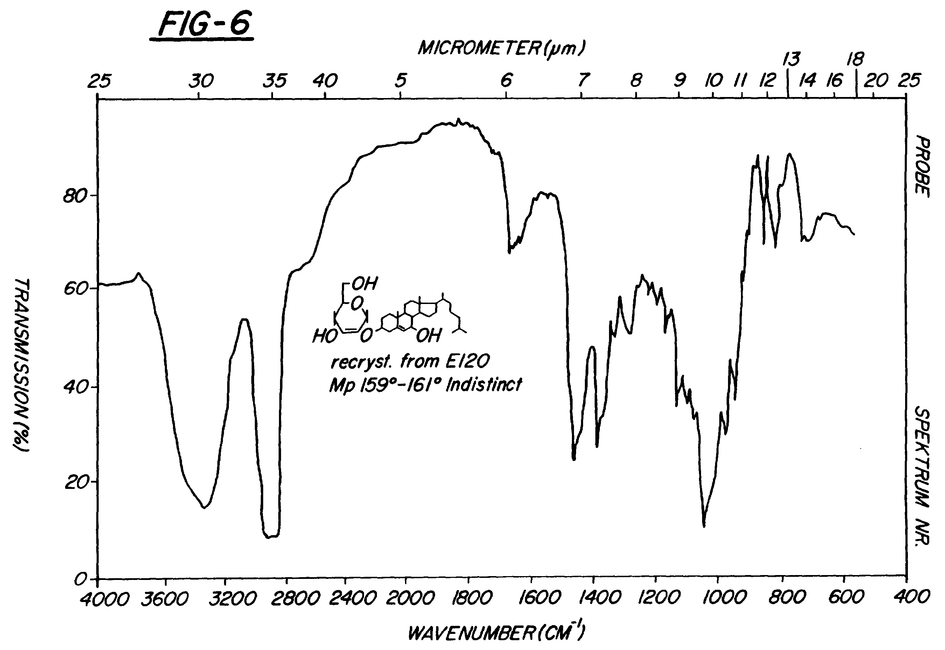

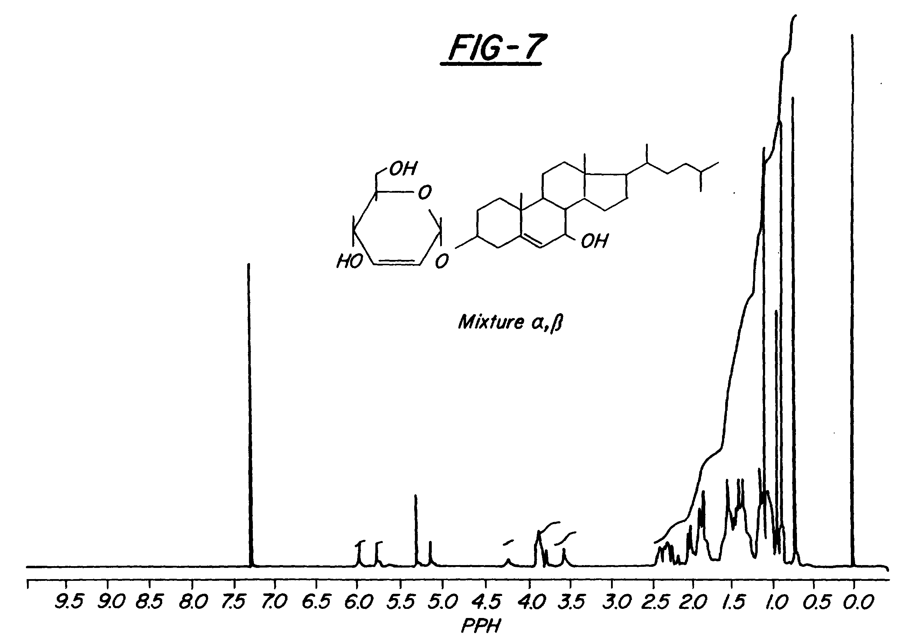

FIGURES 6 and 7 are the IR-spectrum and the NMR-spectrum, respectively, of the 7β-OHC product of Example 3;

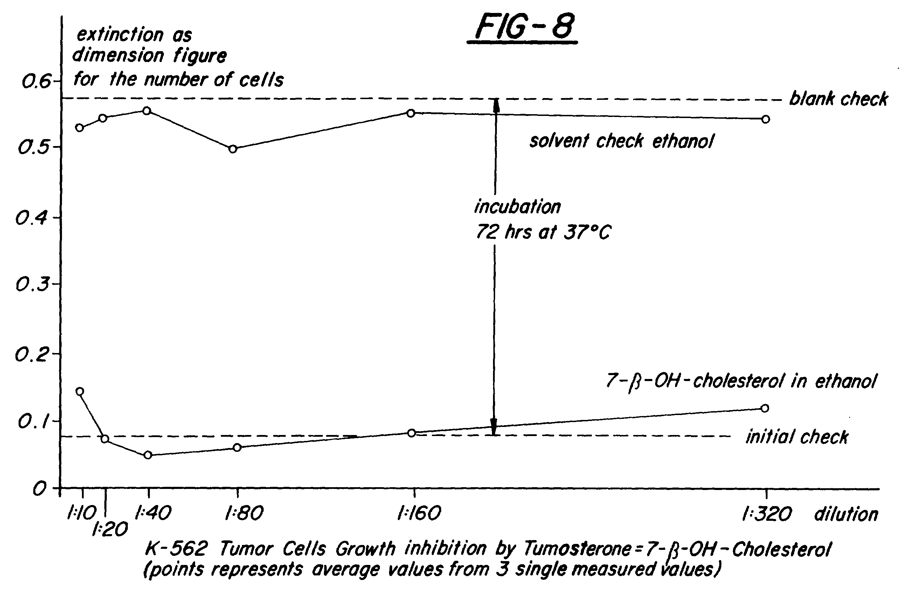

Figure 8 is a plot showing the tumor cell growth inhibition by selected concentrations of 7β-OH cholesterol in cell culture fluid;

According to one preferred embodiment, the invention concerns a method for the production of a pyranoside compound, wherein an aglycon compound selected from aliphatic, alicyclic, aliphatic-aromatic or aromatic compounds having a primary, secondary or tertiary functional group selected from -OH, -SH, and is glycosylated, characterized in that the aglycon compound is reacted in THF with 3,4,6-tri-O-acyl-glucal of formula 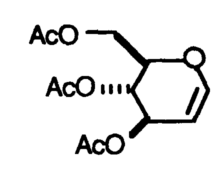 in the presence of molecular or ionized halogen as a catalyst, such as iodine I₂, chlorine Cl₂, bromine Br₂, and fluorine F₂, preferably iodine, to produce the corresponding 4,6-di-O-acyl-2,3-dideoxy-α-D-erythro-hex-2-enopyranoside of said aglycon compound, where acyl is a lower acyl group. In another preferred embodiment a hydroxysteryl compound, preferably a 3β-ol sterol compound, more preferably a delta⁵-3β-ol steroid compound such as a cholesterol, (e.g., delta⁵-cholesten-3β-ol) is glycosylated by reaction with 3,4,6-tri-O-acyl-D-glucal in THF in the presence of molecular or ionized halogen, preferably iodine, as a catalyst. Alternatively, a non-steroidal compound can be thus glycosylated as detailed herein. The reaction is achieved in a single step and in high yield. Thus a double bond which is strongly hindered by the C₄, C₆-acyl groups and thus being inert, is introduced between C₂=C₃ of the glycosidic part of the molecule, whereby as to the hydroxysteryl compound the delta⁵ double bond of the cyclopentano-perhydro-phenanthrene skeleton is stabilized and remains unchanged.

The resulting unsaturated glycoside obtained may be used as an end product or as an intermediate in further reactions to provide functional derivatives which may be steroidal, e.g., cholesterol, derivatives. Thus, in the case of cholesterol, functional groups can be introduced into the perhydrocyclopentano-phenanthrene skeleton of the unsaturated acylglycoside, wherein the α-bond of the acylglycoside at the same time functions as a protecting group for the original OH-group at C₃ of the phenanthrene skeleton.

The present method makes use of iodine which is molecularly dissolved in inert solvents. These inert solvents, for example, comprise CH₂Cl₂ dichloromethane, CHCl₃ chloroform, CCl₄ carbon tetrachloride, C₆H₄(CH₃)₂ xylene, C₆H₃(CH₃)₃ mesitylene, C₆H₅CH(CH₃)₂ cymene, C₆H₁₂ cyclohexane and methyl derivatives thereof, as well as ligroin, petroleum ether and saturated hydrocarbons, such as for example n-pentane or n-heptane, preferably C₆H₆ benzene or C₆H₅CH₃ toluene. Using the alternative solvent nitromethane CH₃NO₂, the glycosylation reaction catalyzed by iodine runs quantitatively at room temperature in 2 hours. The method of the invention for the preparation of the DDH pyranoside also makes use of iodine dissolved in ionizing solvents. Using a ketone such as acetone, methyl ethyl ketone (2-butanone), and cyclohexanone as an alternative solvent, the iodine catalyzed reaction between 3,4,6-Tri-0-acetyl-D-glucal and cholesterol runs quantitatively at 20°C in only 60 to 90 minutes. As a preferred embodiment, one can also use tetrahydrofuran (THF).

Using a lower alcohol such as methanol, ethanol, or propanol, the iodine catalyzed reaction between 3,4,6-Tri-O-acetyl-D-glucal and the aglycon runs during 30 minutes time quantitatively at 20°C as well.

The glycosylation method according to the mentioned preferred embodiment is directed to the reaction of the vinyl ether of 3,4,6-tri-O-acyl-D-glucal with a cholesterol such as delta⁵-cholesten-3β-ol, with molecularly dissolved halogen, i.e. iodine, as catalyst in THF. The reaction thereby introduces a double bond between C-atoms 2 and 3 while eliminating the acyl group sited at C₃, instead of introducing an iodine atom at C₂ in the glycosidic part of the resulting cholesterylglycoside. This reaction is conveniently followed by IR-spectroscopy, and is complete only when the peak of the glucal at 1650 cm⁻¹ has disappeared. The iodine being utilized as catalyst is quantitatively titrated back by a suitable back-titrant reagent such as 0.1 N aqueous sodium thiosulphate (Na₂S₂O₃). The method for providing the corresponding di-O-acyl glycoside as exemplified hereinafter for cholesterol compounds is applicable to the glycosylation of not only steroid compounds and cholesterol compounds and precursors but also aglycon compounds in general, i.e., aliphatic, alicyclic, aliphatic-aromatic and aromatic compounds having a primary, secondary or tertiary functional group of the type mentioned. Aglycon compounds that are useful for their pharmacological properties are preferred, since their respective glycosides produced according to the invention have the same utility and useful properties and art-recognized dosage regimens with the possible enhancement of bioavailability, e.g., at cell membranes, due to the presence of the sugar residue. Preferred hydroxy compounds for glycosylation comprise cholesterols, bile salts, steroid hormones, and vitamin D compounds and precursors as described in Stryer's Biochemistry, 3rd Ed. pp. 559-570, Freeman and Company, New York, 1988. Examples of such compounds are cholic acid and derivatives, 25-hydroxy-cholesterol, 25-hydroxy-calciferol, pregnenolone, 17α-hydroxy-progesterone, 17α-hydroxy-pregnenolone, 11-desoxy-corticosterone, 11-desoxy-cortisol, corticosterone, cortisol, cortisone, dolichol, androsterone, testosterone, estrone, 17β-estradiol, estratriol-3,16α,17β, 3α,5β-tetrahydro-corticosterone, serotonin, urocortisol, and allocortolone, preferably cyclopentanoperhydrophenanthrene compounds having the delta⁵-3β-OH steryl moiety 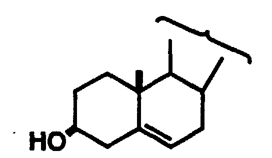 in which the delta⁵ double bond is stabilized as described by 3β-OH glycosylation resulting in the pyranoside.

Other preferred aglycon compounds for glycosylation include
a) morphine and morphine derivatives such as codeine, ethyl morphine, dihydrocodeine, hydromorphone, oxycodone, nalorphine and levorphan;
b) α- and β-sympathomimetic (adrenergic) compounds such as p-hydroxyphenylethanolamine, norfenefrine, synephrine, etilefrin, phenylephrine, octapamine, isoprenaline, dichloroisoproterenol, metaproterenol, terbutaline and buphenine;
c) antihypertensive compounds such as methyldopa;
d) vasoconstrictor compounds such as α-methylnoradrenaline;
e) anticholinergic (parasympatholytic) and antispasmodic compounds such as atropine, homatropine, scopolamine and its methobromide and butyl bromide quaternary compounds, podine methyl sulfate and tropine benzilate;
f) antipsychotic compounds such as acetophenazine, fluphenazine, dixyrazine, perphenazine hydroxyzine, pericyazine, haloperidol, trifluperidol and moperone;
g) cardiotonic compounds such as digitoxigenin, digoxigenin and diginatigenin;
h) estrogen and contraceptive compounds such as ethinylestradiol, mestranol and quinestrol;
i) antibacterial compounds such as amoxicillin, chloramphenicol, thiamphenicol, tetracycline, chlortetracycline and oxytetracycline;
j) antitussive compounds such as chlophedianol, clobutinol and zipeprol;
k) analgesic compounds such as levorphanol, ciramadol, pentazocine, carbetidine, glafenine and salicylamide;
l) oxytocic compounds such as ergonovine, prostaglandin F_{2α};
m) antineoplastic compounds such as fluorouracil;
n) antimalarial compounds such as quinine;
o) fungicidal compounds such as cycloheximide;
p) antirheumatic compounds such as oxyphenbutazone;
q) anticholelithogenic compounds such as chenodiol;
r) choleretic compounds such as hymecromone;
s) anti-gout compounds such as allopurinol;
t) anti-parkinsonian compounds such as levodopa, carbidopa and droxidopa;
u) anti-spasmodic compounds such as ephedrine;
v) nasal decongestant compounds such as cafaminol;
w) muscle relaxant compounds such as phenprobamate and guaiacol glycerol ether;
x) anti-inflammatory compounds such as dexamethasone and beclomethasone; and
y) vitamin and vitamin related compounds such as provitamin D, xanthophyll, vitamin A, vitamin E, thiamin and ascorbic acid.

Other preferred aglycon compounds for glycosylation according to the invention include those listed in the "Therapeutic Category and Biological Activity Index" of The Merck Index XI, pp. THER-5 to THER-29, Merck & Co., Inc., Rahway, NJ.

The glycosylation method and related oxidation and reduction methods described hereinafter may be illustrated by a preferred embodiment employing the starting material cholesterol, as follows:

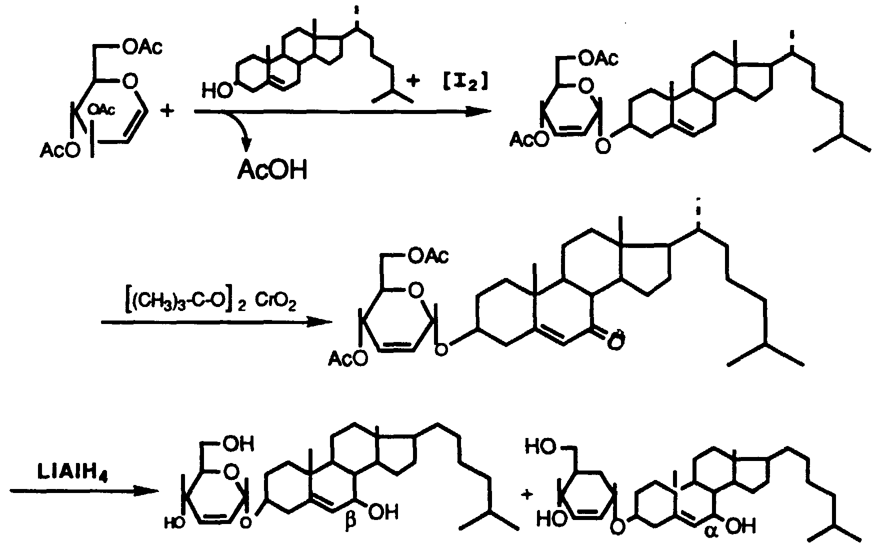

The steryl DDH pyranoside product obtained by the glycosylation method can be converted by oxidation of the steroid part into an α-glycosylated 7-keto-sterol such as α-glycosylated 7-keto-cholesterol. The method is applicable to the oxidation of sterol compounds broadly, preferably cyclopentanoperhydro-phenanthrene compounds having the delta⁵-3β-OH steryl 4,6-di-O-acyl-2,3-dideoxy-α-D-erythro-hex-2-enopyranoside moiety 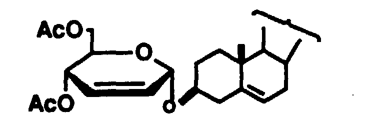 to provide the corresponding 7-keto sterols having the corresponding moiety 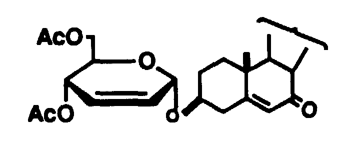 The oxidation is accomplished with an oxidizing agent, which preferably contains chromium, with pyridine-chromium trioxide (C₅H₅N)₂CrO₃ or pyridine-chlorochromate (C₅H₅NHCrO₃)Cl being preferred and t-butyl chromate being especially preferred. The inert glycosidic double bond between C₂=C₃ thereby remains intact as it is shielded by the C₄, C₆ acyl (e.g., acetyl) groups. The reduction of this 7-ketone with a suitable reducing agent, preferably a complex metal hydride, such as one or more of LiAlH₄, NaBH₄, and KBH₄, more preferably LiAlH₄, leads to a steroidal glycoside. In a preferred embodiment, the method importantly provides 7β-hydroxycholesteryl 2,3-dideoxy-α-D-erythro-hex-2-enopyranoside (7β-OHC) of formula 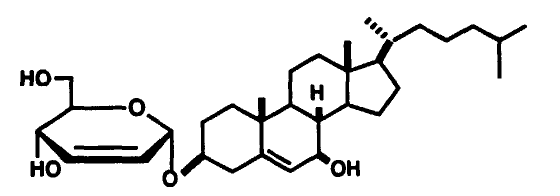 which like cholesterol is systemically biocompatible. The product is obtained after workup of the reaction mixture, e.g. by chromatographic separation of the C₇ β-hydroxy isomer from the C₇ α-hydroxy isomer, in a suitable solvent mixture, preferably a mixture comprising dichloromethane:acetone preferably in 1:1 mixture.

The novel 7β-hydroxycholesterol DDH pyranoside in particular and its 7-keto precursor possess valuable pharmacological properties. For example, a preferred parenteral dosage regimen in treating the proliferative phase growth of the kind described, allowing for ethical considerations and practices exercised in the clinician's judgment, calls for administration of about 10 to about 40 mg. of 7β-OHC DDH pyranoside per 70 Kg. of body weight, once a day or less often while analysis is made of tumor markers such as CEA, TPA, etc. so that the dosage can be adjusted from time to time to normalize the tumor marker level. Whereas the alpha-isomer, delta⁵-cholesten-3β,7α-diol, is formed in the liver as the first degradation product of cholesterol and possesses no physiological activity, the beta-isomer, delta⁵-cholesten-3β,7β-diol (as well as its 7-keto analog), is formed in the thymus gland of all mammals as a universal signal substance of the mammalian immune defense. It owes its activity, which is solely directed to malignant cell surfaces, to the fact that it is bound unspecifically by LDL (low density lipoproteins).

The novel steroid and non-steroid compounds can be used in the form of pharmaceutical preparations comprising each such compound in a pharmacogically effective amount in admixture with a pharmaceutically acceptable carrier which may be conventional per se. These preparations may be formulated by well known procedures. In these respects, see for example *Remington's Pharmaceutical Sciences*, Chapter 43, 14th Ed., Mack Publishing Co., Easton, PA 18042, USA. These preparations can be administered in any suitable way such as orally, e.g. in the form of tablets, dragees, gelatin capsules, soft capsules, solutions, emulsions or suspensions or parenterally, e.g. in the form of injectable solutions at suitable pH, e.g. ca. 7.5, or topically, e.g. in the form of a cream.

The invention and the best mode for practicing the same are illustrated by the following examples.

### Example 1

### Preparation of Cholesteryl 4,6-Di-O-acetyl-2,3-dideoxy-α-D-erythro-hex-2-enopyranoside

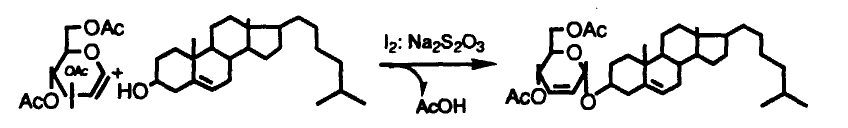

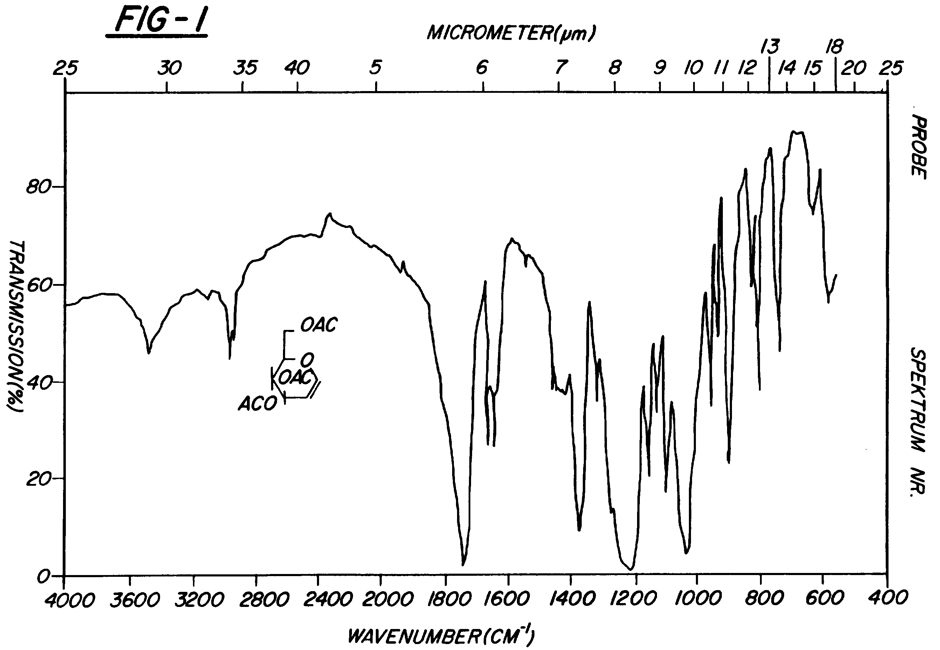

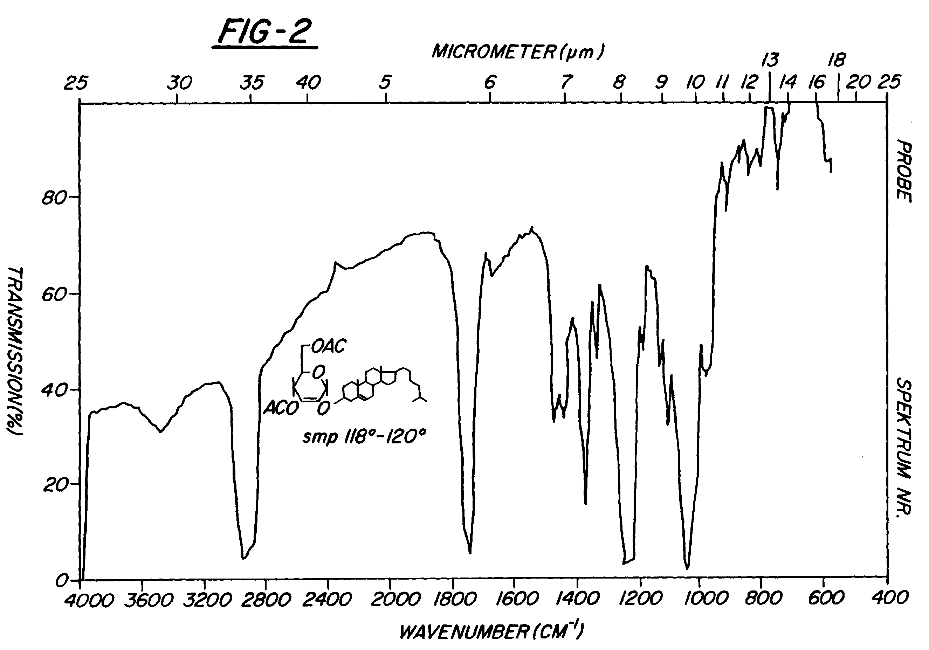

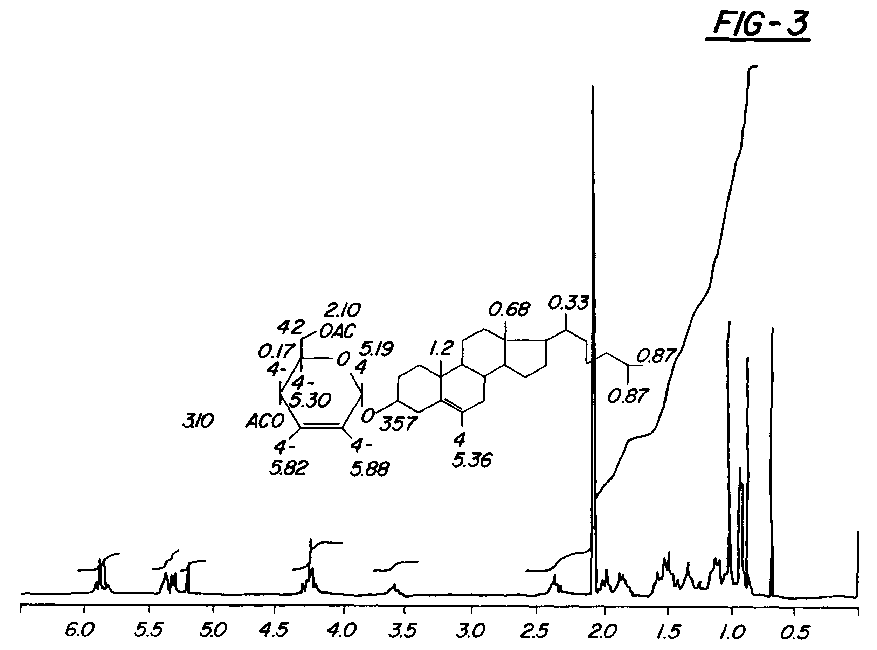

5.0 g (=0.02 mole) molecular iodine were dissolved with stirring in 300 ml benzene in a 2-litre three-necked flask fitted with stirrer, reflux condenser and thermometer. To the wine-red solution thus obtained was added the solution of 27.2 g (=0.10 mole) 3,4,6-tri-O-acetyl-D-glucal and 38.6 g (=0.10 mole) cholesterol (delta⁵-cholesten-3β-ol) in 700 ml of benzene. In the course of 2 hours the mixture was heated to 70-75°C. The reaction was monitored by IR-spectroscopy; it was terminated only when the peak of the glucal at 1650 cm⁻¹ (Figure 1) had disappeared. The red color of the reaction solution is not significant. After removal of the flask heater the reaction solution is rapidly cooled in a water-bath to about 20-30°C. After transfer into a 2-litre separatory funnel the cooled wine-red reaction solution was thoroughly shaken until complete discoloration with 500 ml + 10% of 0.1 N = 12.5 g + 10% = 13.8 g aqueous solution of Na₂S₂O₃, washed twice with water, treated with activated carbon, dried over anhydrous Na₂SO₄ and the solvent distilled off, finally in vacuo.
**Crude yield**: 58.3 g (= 97.4% th.)
   The product, cholesteryl 4,6-di-O-acetyl-2,3-dideoxy-α-D-erythro-hex-2-enopyranoside, is recrystallized from 2 litres of CH₃OH.
**Yield**: 56.95 g (= 95.1% th.)
**Mp**: 118-120°C
**IR-spectrum**: Figure 2
**NMR-spectrum**: Figure 3

The invention comprises not only the above glycoside product but also other glycoside products made by the above procedure using as starting materials equivalent amounts of the glucal (or other suitable glucal) and the aglycon selected for glycosidation. Suitable aglycon compounds are selected (for their known use as aglycons) from the aforementioned aliphatic, alicyclic, aliphatic-aromatic or aromatic compounds having a primary, secondary or tertiary functional group (i.e., -OH, -SH,), preferably a compound listed in the foregoing paragraphs a) to y) or in pages THER-5 to THER-29 of the Merck Index XI, supra. Especially preferred glycoside products made by the above procedure are the respective 4,6-di-O-acyl or acetyl-2,3-dideoxy-α-D-erythro-hex-2-eno-pyranosides of the following aglycon compounds which as DDH pyranoside products are characterized by known IR and NMR spectroscopy data available from the referenced literature.

| Hydroxy Aglycon | The Merck Index IX Monograph No. | Use |
|---|---|---|
| Cholic acid | 2206 | choleretic |
| Clavulanic acid | 2342 | antibacterial |
| Amoxicillin | 610 | antibacterial |
| Daunorubicin | 2825 | antineoplastic |
| Lovastatin | 5460 | antihyper cholesterolemic |
| Mevastatin | 6088 | antihyper cholesterolemic |
| Simvastatin | 8491 | antihyperlipidemic |

The above glycosylation reaction carried out in benzene at 70°C is selective for the desired alpha isomer rather than the beta isomer. Thus the reaction typically produces a ratio of alpha to beta cholesteryl glycosylate isomers (α:β) of greater than 20:1. Similarly, in THF at 67°C and at room temperature the reaction in either case typically produces an α:β ratio of about 9:1.

Another general procedure was used in which, instead of cholesterol, a starting material (i.e., a substrate) having at least one functional group (e.g., OH, SH, and/or -COOH) was used. The procedure is detailed as follows:

### GENERAL PROCEDURE

The substrate (1 equiv) was added to a stirring solution of glucal (0.5-1.0 g) and iodine (20 mol%) in THF at room temperature. The reaction was monitored by thin-layer chromatography. Reaction times are listed for each compound. Upon completion the solution was diluted with ether and washed with 10% aqueous Na₂S₂O₃ solution. The layers were separated and the aqueous layer was extracted with additional ether. The combined organic layers were dried over Na₂S₂O₄, concentrated, and purified by column chromatography on 230-400 mesh SiO₂. All products were recovered as viscous oils except for those compounds for which a melting point is given.
Note: The thiols were added at lower temperatures as indicated for each adduct.

### ILLUSTRATIVE SUBSTRATES

ALCOHOLS (all over 90% yield; α-isomer:β-isomer = 5-8:1)
   methanol
   ethanol
   isopropanol
   tert-butyl alcohol
   benzyl alcohol
   allyl alcohol
PHENOLS (α-isomer:β-isomer = 6 - 8:1)
   phenol (70%)
   *p*-methoxyphenol (ca. 65%)
   *p*-nitrophenol (ca. 60%)
THIOLS (highly reactive)
   - ethyl mercaptan (-78°C to -55°C) (35%): α-isomer:β-isomer = 16:1
   - isopropyl mercaptan (-78°C to 0°C) (77%): α-isomer:β-isomer = 15:1
   - *tert*-butyl mercaptan (-25°C) (68%): α-isomer only
   - thiophenol (room temperature) (50%): α-isomer:β-isomer = 20:1
CARBOXYLIC ACID
   - acetic acid (catalytic) (80%): α-isomer:β-isomer = 10:1

### SPECTROSCOPIC DATA FOR ALCOHOL (ROH) PHENOL (ArOH) AND CARBOXYLIC (RC00H) ADDUCTS

R = Me (Reaction Time = 30 min. Yield = 87%)
   ¹H NMR (CDCl₃; 300 MHz) δ 2.087 (s,3H), 2.111 (s,3H), 3.457 (s,3H), 4.05-4.13 (m,1H), 4.188 (1H) and 4.266 (1H) (ABq,J_{AB}=12.1 Hz; the 4.188 peaks are further split into d with J=2.5 Hz; the 4.266 peaks are further broken down into d with J=5.3 Hz), 4.934 (s,1H), 5.322 (dd,1H,J=9.7, 1.4 Hz), 5.834 (1H) and 5.894 ppm (1H) (ABq,J_{AB}=10.6 Hz; the 5.834 peaks are further split into dd with J=2.0, 2.0 Hz).
   ¹³C NMR (CDCl₃; 75.5 MHz) δ 20.56, 55.70, 63.26, 65.75, 67.35, 95.60, 128.15, 129.38, 170.08, 170.36 ppm.
   IR (KBr) 1048, 1072, 1107, 1137, 1148, 1188, 1233, 1372, 1438, 1449, 1456, 1744, 2910, 2939, 2956 cm⁻¹.
R = Et (Reaction Time = 50 min. Yield = 97%) m.p. (hexanes = 76-77.5°C.
   ¹H NMR (CDCl₃; 300 MHz) δ 1.256 (d,J=7.1 Hz,3H), 2.084 (s,3H), 2.105 (s,3H), 3.584 (1H) and 3.837 (1H) (ABq, J_{AB}=9.7 Hz; both sets of peaks are further split into q with J=7.1 Hz), 4.09-4.14 (m,1H), 4.174 (1H) and 4.261 (1H) (ABq, J_{AB}=12.2 Hz; the 4.174 peaks are further split into dd with J=2.8, 2.8 Hz; the 4.261 peaks are further split into d with J=5.3 Hz), 5.050 (s,1H), 5.319 (dd,J=9.6,1.4 Hz, 1H), 5.837 (1H) and 5,891 ppm (1H) (ABq, J_{AB}=10.2 Hz; the 5.837 peaks are further split into dd with J=1.7, 1.4 Hz).
   ¹³C NMR (CDCl₃; 75.5 MHz) δ 15.31, 20.66, 20.83, 63.30, 64.25, 65.75, 67.25, 94.40, 128.37, 129.13, 170.17, 170.53 ppm.
   IR (KBr) 1019, 1052, 1084, 1108, 1120, 1133, 1137, 1189, 1229, 1239, 1257, 1274, 1372, 1382, 1738, 2902, 2980 cm⁻¹.
R = i-Pr (Reaction Time = 65 min. Yield = 96%)
   ¹H NMR (CDCl₃; 300 MHz) δ 1.186 (d,J=6.2 Hz,3H), 1.258 (d,J=6.2 Hz,3H), 2.081 (s,3H), 2.096 (s,3H), 3.988 (dq,J=6.2, 6.2 Hz,1H), 4.12-4.28 (m,3H), 5.133 (s,1H), 5.301 (dd,J=9.5, 1.4 Hz,1H), 5.804 (1H) and 5.875 ppm (1H) (ABq, J_{AB}=10.3 Hz; the 5.804 peaks are further split into dd with J=2.6, 2.5 Hz,1H).
   ¹³C NMR (CDCl₃; 75.5 MHz) δ 20.59, 20.83, 22.06, 23.54, 63.42, 65.87, 67.22, 70.83, 93.06, 128.82, 128.91, 170.18, 170.55 ppm.
   IR (KBr) 1035, 1101, 1125, 1185, 1231, 1372, 1457, 1744, 2933, 2973 cm⁻¹.
R = t-Bu (Reaction Time = 2 hr. Yield = 74%)
   ¹H NMR (CDC)₃: 300 MHz) δ 1.292 (s,9H), 2.075 9s,3H), 2.085 (s,3H), 4.12-4.28 (m,3H), 5.271 (d,1H,J=8.0 Hz), 5.321 (dd,1H,J=1.3, 1.3 Hz), 5.744 (1H), and 5.844 ppm (1H) (ABq,J_{AB}=10.2 Hz; the 5.744 peaks are further split into dd with J=2.8, 2.8 Hz).
   ¹³C NMR (CDCl₃; 75.5 MHz) δ 20.72, 20.93, 28.79, 63.35, 65.49, 66.72, 75.23, 89.02, 128.13, 129.67, 170.17, 170.57 ppm.
   IR (KBr) 1025, 1044, 1100, 1183, 1196, 1235, 1370, 1392, 1746, 2935, 2976 cm⁻¹.
R = Ph (Reaction Time = 8 hr. Yield = 70%)
   ¹H NMR (CDCl₃; 300 MHz) δ 1.976 (s,3H), 2.017 (s,3H), 4.10-4.30 (m,3H), 5.387 (d,J=11.4 Hz,1H), 5.698 (s,2H), 7.00-7.33 ppm (m,5H).
   ¹³C NMR (CDCl₃; 75.5 MHz) δ 20.54, 20.84, 62.66, 65.13, 67.85, 92.12, 117.10, 122.42, 127.14, 129.40, 130.06, 157.08, 170.06, 1740.46 ppm.
   IR (KBr) 1005, 1029, 1047, 1076, 1096, 1187, 1222, 1371, 1491, 1589, 1598, 1744 cm⁻¹.
R = p-MeO-Ph (Reaction Time = 12 hr. Yield = 75%) m.p. (hexanes) = 77-77.5°C.
   ¹H NMR (CDCl₃; 300 MHz) δ 2.023 (s,3H), 2.109 (s,3H), 3.774 (s,3H), 4.14-4.30 (m,3H), 5.376 (d,J=9.1 Hz,1H), 5.567 (s,1H), 6.007 (s,1H), 6.830 (2H) and 7.047 ppm (2H) (ABq,J_{AB}=9.0 Hz).
   ¹³C NMR (CDCl₃; 75.5 MHz) δ 20.61, 20.85, 55.77, 63.03, 65.60, 67.94, 94.23, 114.85, 115.11, 116.21, 118.90, 127.51, 130.08, 170.32, 170.75 ppm.
   IR (KBr) 1010, 1037, 1053, 1184, 1227, 1243, 1255, 1270, 1380, 1507, 1741, 2922, 2934, 2958 cm⁻¹.
R = CH₂Ph (Reaction Time = 80 min. Yield = 95%)
   ¹H NMR (CDCl₃; 300 MHz) δ 2.081 (s,3H), 2,102 (s,3H), 4.09-4.29 (m,3H), 4.601 (1H) and 4.809 (1H) (ABq,J_{AB}=11.7 Hz), 5.139 (s,1H), 5.334 (dd,J=9.4, 1.3 Hz,1H) 5.875 (ABq,2H), 7.29-7.40 ppm (m,5H).
   ¹³C NMR (CDCl₃; 75.5 MHz) δ 20.63, 20.81, 63.19, 65.74, 67.50, 70.35, 93.83, 127.00, 128.56, 128.56, 129.46, 170.17, 170.59 ppm.
   IR (KBr) 1026, 1038, 1101, 1137, 1151, 1186, 1227, 1370, 1405, 1436, 1454, 1743, 2904, 2937, 2953, 3032 cm⁻¹.
R = CH₂CH=CH₂ (Reaction Time = 80 min. Yield = 88%)
   ¹H NMR (CDCl₃; 300 MHz) δ 2.086 (s,3H), 2.106 (s,3H), 4.05-4.30 (m,5H), 5.084 (s,1H), 5.212 (d,J=10.3 Hz, 1H), 5.28-5.35 (m,2H), 5.848 (1H) and 5.899 (1H) (ABq,J_{AB}=10.3 Hz), 5.92-5.99 ppm (m,1H).
   ¹³C NMR (CDCl₃; 75.5 MHz) δ 20.73, 20.91, 63.00, 65.35, 67.05, 69.22, 93.65, 117.35, 127.86, 129.27, 134.27, 170.18, 170.62 ppm.
R = CH₂C(CH₃)₃ (Reaction Time = 30 min. Yield = 97%)
   ¹H NMR (CDCl₃; 300 MHz) δ 0.931 (s,9H), 2.086 (s,3H), 3.133 and 3.481 (ABq,J_{AB}=8.8 Hz, 2H), 4.07-4.27 (m,3H), 4.985 (s,1H) 5.305 (d,J=9.5 Hz,1H), 5.850 and 5.859 (ABq,J_{AB}=10.5 Hz, 2H; the 5.850 ppm peaks are further split into dd with J=2.7 and 2.6 Hz).
   ¹³C NMR (CDCl₃; 75.5 MHz) δ 20.73, 20.91, 63.00, 65.35, 67.05, 69.22, 93.65, 117.35, 127.86, 129.27, 134.27, 170.18, 170.62 ppm.
R = OAc (Reaction time 24 h. Yield = 81%) (20 mol% of acetic acid was used).
   ¹H NMR (CDCl₃; 300 MHz) δ 2.082 (s,3H), 2.095 (s,3H), 2.098 (s,3H), 4.06-4.28 (m,3H), 5.368 (d,J=10.5 Hz, 1H), 5.850 and 6.011 (ABq, J_{AB}=10.6 Hz, 2H; the 5.850 ppm peaks are further split into dd with J=2.7 and 2.2 Hz), 6.294 ppm (s,1H).

### SPECTROSCOPIC DATA FOR THIOL (RSH) ADDUCTS

R = Et (-78 to -55°C, Reaction Time = 3.5 hr. Yield = 35%)
   ¹H NMR (CDCl₃; 300 MHz) δ 1.252 (t,J=7.1 Hz, 3H), 2.080 (s,3H), 2.099 (s,3H), 3.581 (1H) and 3.832 (1H) (ABq,J_{AB}=9.7 Hz; both sets of peaks are further split into dq with J=7.1, 7.1 Hz), 4.08-4.29 (m,3H), 5.043 (s,1H), 5.315 (dd,J=9.6, 1.3 Hz, 1H), 5.831 (1H) and 5.883 ppm (1H) (ABq,J_{AB}=10.3 Hz).
   ¹³C NMR (CDCl₃; 75.5 MHz) δ 15.31, 20.72, 20.92, 63.15, 64.30, 65.52, 67.02, 94.30, 128.09, 129.03, 170.20, 170.66 ppm.
   IR (KBr) 1049, 1079, 1182, 1232, 1371, 1437, 1451, 1743, 2930, 2964 cm⁻¹.
R = i-Pr (-78 to 0°C, Reaction Time = 6 hr. Yield = 77%)
   ¹H NMR (CDCl₃; 300 MHz) δ 1.337 (d,3H,J=6.8 Hz), 1.342 (d,3H,J=6.8 Hz), 2.085 (s,6H), 3.145 (dq,1H,J=6.8, 6.8 Hz), 4.15-4.38 (m,3H), 5.349 (d,1H,J=9.6 Hz), 5.638 (s,1H), 5.761 (1H) and 5.935 ppm (1H) (ABq,J_{AB}=10.1 Hz).
   ¹³C NMR (CDCl₃; 75.5 MHz) δ 20.66, 20.92, 23.88, 36.3, 63.14, 65.25, 66.93, 79,61, 126.74, 129.39, 170.21, 170.60 ppm.
   IR (KBr) 1050, 1078, 1122, 1157, 1181, 1229, 1369, 1419, 1452, 1743, 2868, 2927, 2960 cm⁻¹.
R = t-Bu (-25 to 0°C, Reaction Time = 2 hr. Yield = 68%)
   ¹H NMR (CDCl₃; 300 MHz) δ 1.404 (s,9H), 2.070 (s,3H), 2.080 (s,3H), 4.13-4.37 (m,3H), 5.325 (dd,1H,J=9.3, 1.8 Hz), 5.739 (d,1H,J=1.7 Hz), 5.753 (1H) and 5.902 ppm (1H) (ABq,J=10.9 Hz).
   ¹³C NMR (CDCl₃; 75.5 MHz) δ 20.70, 20.94, 31.51, 44.33, 63.22, 65.16, 66.84, 78.17, 126.61, 129,67, 170.30, 170.72 ppm.
   IR (KBr) 1054, 1078, 1164, 1236, 1368, 1744, 2962 cm⁻¹.

By the same procedure, but using mevinolin (lovastatin) as a substrate rather than cholesterol, the resulting adduct in about 60% yield is Lovastatin 4,6-di-O-acetyl-2,3-dideoxy-α-D-erythro-hex-2-enopyranoside having the formula: 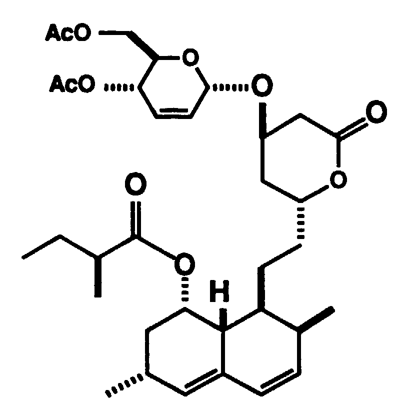 The reaction is carried out under mild conditions, e.g., preferably at room temperature, for one hour, using triacetyl D-glucal (1.0 equiv), iodine (20 mol %) in THF as the solvent.
R = Lovastatin (Reaction Time = 1 h. Yield = 55%)
   ¹H NMR (CDCl₃; 300 MHz) δ 0.873 (t,J=7.5 Hz,3H), 0.892 (d,J=7.0 Hz, 3H), 1.074 (d,J=7.4 Hz, 3H), 1.105 (d,J=6.9 Hz, 3H), 2.091 (s,3H), 2.094 (s,3H), 3.99-4.05 (m,1H), 4.14-4.28 (m,3H), 4.29-4.34 (m,1H), 4.52-4.61 (m,1H), 5.134 (s,1H), 5.323 (dd,J=9.6, 1.3 Hz, 1H), 5.373 (d,J=2.7 Hz, 1H), 5.75-5.91 (m,2H), 5.909 (part of ABq, J_{AB}=11.1 Hz, 1H), 5.992 ppm (d,J=9.7 Hz, 1H).
   ¹³C NMR (CDCl₃; 75.5 MHz) δ 11.67, 13.92, 16.23, 20.70, 20.88, 22.85, 24.23, 26.79, 27.51, 30.77, 32.77, 33.22, 35.01, 35.73, 36.65, 37.46, 41.45, 62.98, 65.26, 67.38, 67.85, 68.65, 76.70, 93.12, 127.44, 128.44, 129.49, 129.72, 131.65, 132.94, 169.32, 170.07, 170.47, 176.39 ppm.

By the same procedure, but using either mephenesin or capaicin as a substrate rather than mevinolin, the resulting adduct is mephenesin 4,6-di-O-acetyl-2,3-dideoxy-α-D-erythro-hex-2-enopyranoside and capsaicin 4,6-di-O-acetyl-2,3-dideoxy-α-D-erythro-hex-2-enopyranoside, respectively.
R = Mephenesin (Reaction Time = 1 h. Yield = 85%)
   ¹H NMR (CDCl₃; 300 MHz) δ 2.068 (s,3H), 2.071 (s,3H), 2.077 (s,3H), 2.080 (s,3H), 2.214 (brs, 3H), 3.70-3.82 (m,3H), 3.99-4.40 (m,11H), 5.06-5.12 (m,1H), 5.28-5.39 (m,3H), 5.78-5.99 (m,4H), 6.81-6.91 (m,3H), 7.11-7.19 ppm (m,2H).
R = Capsaicin (Reaction Time = 8 h. reflux in THF. Yield = 60%)
   ¹H NMR (CDCl₃; 300 MHz) δ 0.834 (d,J=6.6 Hz, 3H), 0.948 (d,J=6.7 Hz, 3H), 2.091 (s,3H), 2.093 (s,3H), 3.850 (s), 5.27-5.35 (m,2H), 5.45-5.90 (m,4H), 6.71-6.88 (m,3H).

Adducts with cholic acid or a cholic acid analog by this procedure are as follows:

### Chenodeoxycholic Acid

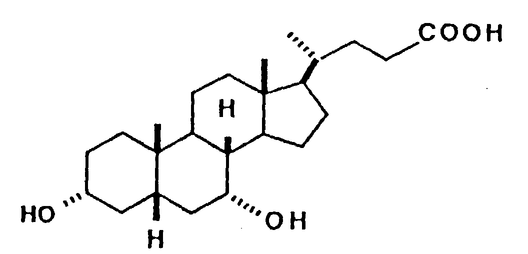

Conditions: triacetyl D-glucal (2.0 equiv); iodine (20 mol %); THF, room temperature, 4h
Results: diadduct (89%) chenodeoxycholic acid 3,12-di-(4,6-di-O-acetyl-2,3-deoxy-α-D-erythro-hex-2-enopyranoside).

### Deoxycholic Acid

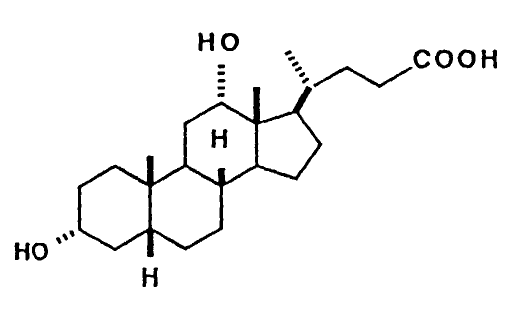

Conditions: triacetyl D-glucal (2.0 equiv); iodine (20 mol %); THF, room temperature, 4h
Results: diadduct (91%) chenodeoxycholic acid 3,12-di-(4,6-di-O-acetyl-2,3-deoxy-α-D-erythro-hex-2-enopyranoside).

### Cholic Acid

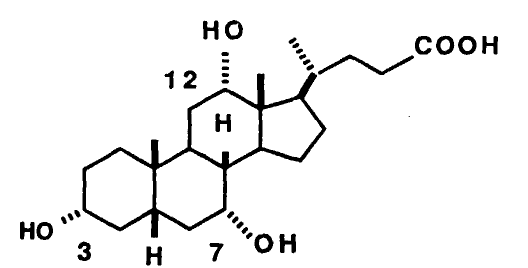

Conditions: triacetyl D-glucal (3.0-5.0 equiv); iodine (20 mol %); THF, reflux, 8h
   - Results:: mixture of di- and triadducts (85%):
   cholic acid 3,7-di-(DDH pyranoside);
   cholic acid 3,12-di-(DDH pyranoside); and
   cholic acid 3,7,12-tri-(DDH pyranoside).

An adduct with chloramphenicol by this procedure is as follows:

### Chloramphenicol

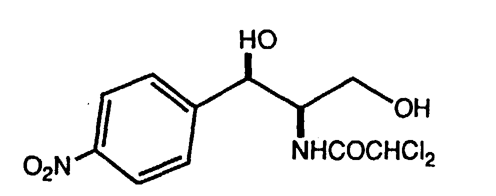

Conditions: triacetyl D-glucal (2.0 equiv); iodine (20 mol %); THF, room temperature, 6h
Results: diadduct (85%) chloramphenicol 1,3-di-(DDH pyranoside).

Having described the invention, the embodiments of the invention in which an exclusive property or privilege is claimed are defined as follows:

## Claims

1. Method for the production of a pyranoside compound, wherein a aglycon compound selected from aliphatic, alicyclic, aliphatic-aromatic and aromatic compounds having a primary, secondary or tertiary functional group selected from -SH and -COOH is glycosylated, characterized in that the aglycon compound is reacted in a solvent with 3,4,6-tri-O-acyl-glucal of formula 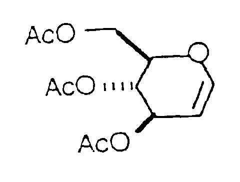 in the presence of molecular halogen preferably molecular iodine as a catalyst to produce the corresponding 4,6-di-O-acyl-2,3-dideoxy-α-D-erythro-hex-2-enopyranoside of said aglycon compound, where acyl is a lower acyl group.

2. Method according to claim 1, characterized in that benzene or toluene is used as the solvent.

3. Method for the production of a pyranoside compound, wherein a aglycon compound selected from aliphatic, alicyclic, aliphatic-aromatic and aromatic compounds having a primary, secondary or tertiary functional group selected from -OH, -SH and -COOH is glycosylated, characterized in that the aglycon compound is reacted in tetrahydrofuran with 3,4,6-tri-O-acyl-glucal of formula 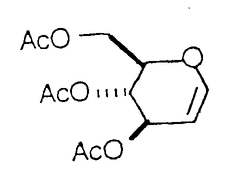 in the presence of molecular halogen preferably molecular iodine as a catalyst to produce the corresponding 4,6-di-O-acyl-2,3-dideoxy-α-D-erythro-hex-2-enopyranoside of said aglycon compound, where acyl is a lower acyl group.

4. Method according to claim 3, characterized in that the aglycon compound is a delta⁵-3β-ol steryl compound.

5. Method according to claim 4, characterized in that a cholesterol compound is reacted with 3,4,6-tri-O-acetyl-D-glucal in a solvent to yield the corresponding 4,6-di-O-acetyl-2,3-dideoxy-α-D-erythro-hex-2-enopyranoside.

6. Method according to claim 5, characterized in that the 4,6-di-O-acetyl-2,3-dideoxy-α-D-erythro-hex-2-enopyranoside is oxidized with an oxidizing agent to produce 7-ketocholesteryl 4,6-di-O-acetyl-2,3-dideoxy-α-D-erythro-hex-2-enopyranoside which in turn is reduced with a metal hydride as a reducing agent to produce 7-β-hydroxycholesteryl 2,3-dideoxy-α-D-erythro-hex-2-enopyranoside.

7. Method according to claim 6 characterized in that the oxidizing agent is selected from the group consisting essentially of t-butyl chromate, pyridine-chromium trioxide, and pyridine chlorochromate.

8. Method according to claim 6, characterized in that one or more of LiAlH4, NaBH₄, and KBH₄ is used as a reducing agent.

9. Method according to claim 6, characterized in that the 7β-hydroxycholesteryl pyranoside is isolated by chromatography.

10. Method according to claim 9, characterized in that the isolation is carried out by chromatography using a solvent mixture which comprises dichloromethane and acetone.

## Patentansprüche

1. Verfahren zur Herstellung einer Pyranosidverbindung, wobei eine Aglykonverbindung, ausgewählt aus aliphatischen, alicyclischen, aliphatisch-aromatischen und aromatischen Verbindungen, mit einer primären, sekundären oder tertiären funktionellen Gruppe, ausgewählt aus -SH und -COOH, glykosyliert wird, dadurch gekennzeichnet, daß die Aglykonverbindung in einem Lösungsmittel mit 3,4,6-Tri-O-acyl-glucal der Formel 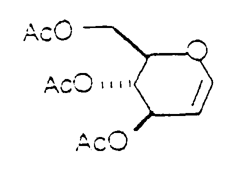 in Anwesenheit von molekularem Halogen, vorzugsweise molekularem Iod. als Katalysator umgesetzt wird, um das entsprechende 4,6-Di-O-acyl-2,3-didesoxy-α-D-erythro-hex-2-enopyranosid der Aglykonverbindung, wobei Acyl eine niedere Acylgruppe ist, herzustellen.

2. Verfahren gemäß Anspruch 1, dadurch gekennzeichnet, daß Benzol oder Toluol als Lösungsmittel verwendet wird.

3. Verfahren zur Herstellung einer Pyranosidverbindung, wobei eine Aglykonverbindung, ausgewählt aus aliphatischen, alicyclischen, aliphatisch-aromatischen und aromatischen Verbindungen, mit einer primären. sekundären oder tertiären funktionellen Gruppe, ausgewählt aus -OH, -SH und -COOH, glykosyliert wird, dadurch gekennzeichnet, daß die Aglykonverbindung in Tetrahydrofuran mit 3,4,6-Tri-O-acyl-glucal der Formel 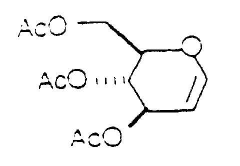 in Anwesenheit von molekularem Halogen, vorzugsweise molekularem Iod, als Katalysator umqesetzt wird, um das entsprechende 4,6-Di-O-acyl-2,3-didesoxy-α-D-erythro-hex-2-enopyranosid der Aglykonverbindung, wobei Acyl eine niedere Acylgruppe ist, herzustellen.

4. Verfahren gemäß Anspruch 3, dadurch gekennzeichnet, daß die Aglykonverbindung eine Delta⁵-3β-ol-sterylverbindung ist.

5. Verfahren gemäß Anspruch 4, dadurch gekennzeichnet, daß eine Cholesterinverbindung mit 3,4,6-Tri-O-acetyl-D-glucal in einem Lösungsmittel umgesetzt wird, um das entsprechende 4,6-Di-O-acetyl-2,3-didesoxy-α-D-erythro-hex-2-enopyranosid zu liefern.

6. Verfahren gemäß Anspruch 5, dadurch gekennzeichnet, daß das 4,6-Di-O-acetyl-2,3-didesoxy-α-D-erythro-hex-2-enopyranosid mit einem Oxidationsmittel oxidiert wird, um 7-Ketocholesteryl-4,6-di-O-acetyl-2,3-didesoxy-α-D-erythro-hex-2-enopyranosid herzustellen, welches dann wieder mit einem Metallhydrid als Reduktionsmittel reduziert wird, um 7-β-Hydroxycholesteryl-2,3-didesoxy-α-D-erythro-hex-2-enopyranosid herzustellen.

7. Verfahren gemäß Anspruch 6, dadurch gekennzeichnet, daß das Oxidationsmittel aus der Gruppe ausgewählt ist, die im wesentlichen aus t-Butylchromat, Pyridin-Chromtrioxid und Pyridinchlorochromat besteht.

8. Verfahren gemäß Anspruch 6, dadurch gekennzeichnet, daß eines oder mehrere von LiAlH₄, NaBH₄ und KBH₄ als Reduktionsmittel verwendet werden.

9. Verfahren gemaß Anspruch 6, dadurch gekennzeichnet, daß das 7β-Hydroxycholesterylpyranosid durch Chromatographie isoliert wird.

10. Verfahren gemäß Anspruch 9, dadurch gekennzeichnet, daß die Isolierung durch Chromatographie ausgeführt wird, wobei ein Lösungsmittelgemisch verwendet wird, das Dichlormethan und Aceton umfaßt.

## Revendications

1. Procédé pour la production d'un composé pyrannoside, dans lequel un composé aglycone choisi parmi les composés aliphatiques, alicycliques, aliphatiques-aromatiques et aromatiques ayant un groupe fonctionnel primaire, secondaire ou tertiaire choisi parmi -SH et -COOH est glycosylé, caractérisé en ce que le composé aglycone est mis à réagir dans un solvant avec un 3,4,6-tri-O-acyl-glucal de formule 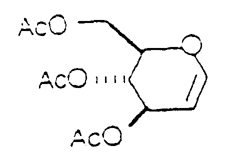 en présence d'un halogène moléculaire, de préférence de l'iode moléculaire, en tant que catalyseur pour produire le 4,6-di-O-acyl-2,3-didésoxy-α-D-érythro-hex-2-énopyrannoside correspondant dudit composé aglycone, dans lequel le groupe acyle est un groupe acyle inférieur.

2. Procédé selon la revendication 1, caractérisé en ce que du benzène ou du toluène est utilisé en tant que solvant.

3. Procédé de préparation d'un composé pyrannoside, dans lequel un composé aglycone choisi parmi les composés aliphatiques, alicycliques, aliphatiques-aromatiques et aromatiques ayant un groupe fonctionnel primaire, secondaire ou tertiaire choisi parmi -OH, -SH et -COOH est glycosylé, caractérisé en ce que le composé aglycone est mis à réagir dans du tétrahydrofuranne avec un 3,4,6-tri-O-acyl-glucal de formule 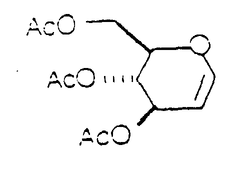 en présence d'un halogène moléculaire, de préférence de l'iode moléculaire. en tant que catalyseur pour produire le 4,6-di-O-acyl-2,3-didésoxy-α-D-érythro-hex-2-énopyrannoside correspondant dudit composé aglycone, dans lequel le groupe acyle est un groupe acyle inférieur.

4. Procédé selon la revendication 3, caractérisé en ce que le composé aglycone est un composé delta⁵-3β-ol stéryle.

5. Procédé selon la revendication 4, caractérisé en ce qu'un composé de cholestérol est mis à réagir avec du 3,4,6-tri-O-acétyl-D-glucal dans un solvant pour donner le 4,6-di-O-acétyl-2,3-didésoxy-α-D-érythro-hex-2-énopyrannoside correspondant.

6. Procédé selon la revendication 5, caractérisé en ce que le 4,6-di-O-acétyl-2,3-didésoxy-α-D-érythro-hex-2-énopyrannoside est oxydé avec un agent oxydant pour produire du 7-cétocholestéryl-4,6-di-O-acétyl-2,3-didésoxy-α-D-érythro-hex-2-énopyrannoside qui est à son tour réduit avec un hydrure de métal en tant qu'agent réducteur pour produire du 7-β-hydroxycholestéryl 2,3-didésoxy-α-D-érythro-hex-2-énopyrannoside.

7. Procédé selon la revendication 6, caractérisé en ce que l'agent oxydant est choisi dans le groupe essentiellement constitué par le chromate de t-butyle, la pyridine-trioxyde de chrome et le chlorochromate de pyridine.

8. Procédé selon la revendication 6, caractérisé en ce qu'un ou plusieurs composés parmi LiAlH₄, NaBH₄ et KBH₄ est utilisé en tant qu'agent réducteur.

9. Procédé selon la revendication 6, caractérisé en ce que le 7β-hydroxycholestéryl pyrannoside est isolé par chromatographie.

10. Procédé selon la revendication 9, caractérisé en ce que l'isolement est effectué par chromatographie en utilisant un mélange de solvants qui comprend du di chlorométhane et de l'acétone.
